# EUROPÄISCHE PATENTSCHRIFT

(11) **EP 1 537 896 B1**
(45) Veröffentlichungstag und Bekanntmachung des Hinweises auf die Patenterteilung: **02.04.2008**
(21) Anmeldenummer: 04026785.8
(22) Anmeldetag: 11.11.2004
(51) Int. Cl.: A61P 3/10, A61K 31/195, A61K 38/06, A61K 31/185, A61K 31/10, A61K 31/7076, A61K 31/375, A61K 31/355, A61K 31/07, A61K 31/095, A61K 31/685, A61K 31/593, A61K 31/51, A61K 31/525, A61K 31/4415, A61K 31/714, A61K 31/565

(54) **Stoffgemische basierend auf schwefelhaltigen Aminosäuren, Antioxidantien, Phospholipiden und Vitaminen zur Herstellung eines Arzneimittels für die Behandlung des L-Diabetes und/oder assoziierter Erkrankungen**
Compound mixtures based on sulphur containing amino acids, antioxidants, phospholipids and vitamins for the preparation of a medicament for treating L-diabetes and/or associated diseases.
Mélanges de substances contenants des acides aminés sulfurés, des antioxidants, des phospholipides et des vitamines pour la préparation d'un médicament pour le traitement du diabète de type L ou/et des maladies associées.

(30) Priorität: 03.12.2003 EP 03027678; 27.01.2004 EP 04001650
(43) Veröffentlichungstag der Anmeldung: 08.06.2005
(73) Patentinhaber: Liebel, Franz-Peter, Dr., 72663 Grossbettlingen (DE)
(72) Erfinder: Liebel, Franz-Peter, Dr., 72663 Grossbettlingen (DE)

(56) Entgegenhaltungen:
- EP-A2- 0 768 043
- WO-A1-03/024487
- WO-A2-98/41113
- US-A1- 2003 078 269
- US-A1- 2003 134 851
- DATABASE WPI Section Ch, Week 200234 Derwent Publications Ltd., London, GB; Class B04, AN 2002-292986 XP002315113 & CN 1 334 097 A (UNIV ANGLI CO LTD SHANGHAI JIAOTONG) 6. Februar 2002 (2002-02-06)

## Beschreibung

### A) Verwendbarkeit der Erfindung

Die Erfindung wird verwendet zur Prophylaxe und Therapie des hier so benannten L-Diabetes und der damit assoziierten Erkrankungen. Der L-Diabetes wird definiert als Teil des nicht-autoimmunopathischen und - nach manifester Erkrankung - nicht-adipösen Diabetes mellitus, der charakterisiert ist durch einen zentralnervös falsch niedrig gemessenen Ist-Wert der Blutglucose. Diese Falschmessung entsteht im ZNS durch die Abnahme der intra-/ extrazellulären ATP-Konzentration, die nicht durch eine Abnahme der Blutglucose verursacht, aber als solche interpretiert wird. Als Ursachen, die über eine kausale Verkettung die Minderung der cerebralen ATP-Bereitstellung bewirken, werden Defekte an Enzymen der UDP-Glucuronosyltransferase 1 (UGT1), eine genetisch bedingte oder erworbene Überempfindlichkeit des sympathischen Systems, eine genetisch bedingte oder erworbene Unterversorgung mit IgM und Reaktionen auf unverträgliche Nahrungsmittel postuliert.
Da bei 8 % der Kaukasier UGT1A1 und damit verknüpft fast immer auch UGT1A6 sowie UGT1A7 jeweils ohne Wildtyp-Allel vorliegen, ist bei diesem Teil der Bevölkerung mit einem entsprechenden Entgiftungsdefizit zu rechnen.
Die verwendeten schwefelhaltigen Aminosäuren erhöhen wirksam die Möglichkeiten zur Sulfatierung, Glutathionkonjugation, Taurinkonjugation und Metallothionein-Synthese, und man erreicht so die Entgiftung von endogenen und exogenen Toxinen. Diese Giftminderung ist bei mangelhafter Glucuronidierung und überbeanspruchtem Sulfatpool von wesentlicher Bedeutung.
Es ist beabsichtigt, die fatalerweise im ZNS sich bildende Hypoglykämie-Falschmeldung, verursacht über den Kausalweg [ATP-Defizit → abnehmende ATP-Registrierung an ATP-sensorischen Kernen → Hypoglykämie-Meldung], zu bessern.

### B) Stand der Technik und Vorteile des Verfahrens

### Dokument D1: US 2003/078269 A1 (PEARSON DON C. ET AL.)

Es handelt sich bei D1 um Stoffgemische, die Biguanide und/oder Sulfonylharnstoffe enthalten, zusammen mit anderen aktiven Ingredientien. Diese sogenannten aktiven Ingredientien sind beigefügt "... to prevent and rectify adverse events associated with insulin resistance syndrome and diabetes mellitus,...". Neben der ganz allgemein gehaltenen Absicht, die Progression der Insulinresistenz hin zum Typ 2 Diabetes zu hemmen, werden konkret als widrige Ereignisse die mikro- und makrovaskulären Komplikationen genannt. Die in D1 genannten aktiven Ingredientien mit Überschneidungen in der hier eingereichten Patentanmeldung sind Ascorbinsäure, Taurin, Folsäure, α-Liponsäure, N- Acetylcystein, Selen, Zink, Thiamin und Tocopherole.

### Fehlende Übereinstimmung:

Das konkurrierende Dokument enthält kein pathogenetisches Kausalkonzept des Diabetes mellitus Typ II, das mit dem Pathogenesekonzept der hier eingereichten Patentanmeldung Übereinstimmungen aufweisen könnte.
Die Stoffgemische des konkurrierenden Dokuments sind an keiner Stelle mit der Stoffkombination identisch, die in der hier eingereichten Patentanmeldung enthalten ist. Vielmehr wird in dem eingereichten Anspruch 1 eine ***Kombination*** aus genau sechs Teilarzneimitteln zusammengestellt. Durch die Kombination entsteht ein neuartiges Arzneimittel, dessen Komponenten gleichzeitig an sechs Stellen des L-diabetischen Pathogenesekonzepts zur Wirkung kommen.
Kennzeichnend ist auch die Verwendung ***injizierbarer Vitaminpräparate**.*
Es ist auszuschließen, daß die hier eingereichte Arzneimittelkombination mit Kombinationspräparaten konkurrierender Patentanmeldungen übereinstimmt. Dies ist eine Folge des *neu aufgestellten und im einzelnen deklarierten Entstehungsablaufs* des L-Diabetes, der ausschließlich die Auswahl der Medikamente bestimmt, so daß Übereinstimmungen mit den Präparatkombinationen anderer Patentanmeldungen oder anderer Veröffentlichungen nicht vorkommen.

### Dokument D2: WO 02/102360 A2 (DIOGUARDI FRANCESCO S. ET AL.)

Es handelt sich um Stoffgemische, die verzweigtkettige Aminosäuren sowie Threonin und Lysin enthalten, "preferably" ergänzt durch Methionin und Cyst(e)in. Die Absicht ist, die myokardiale Ventrikelfunktion bei Diabetespatienten zu verbessern.
Fehlende Übereinstimmung: Ebenso wie in D1.

### Dokument D3: WO 02/13814 A1 (REMACLE CLAUDE ET AL.)

Es handelt sich um Verbindungen, die Schwefel tragen, und dies können Taurin, L-Cystein, L-Methionin oder eine Kombination dieser Stoffe sein.. Die Absicht ist, auf das Inselorgan eine anti-apoptotische und/oder immunmodulatorische Einwirkung zu erzielen
Fehlende Übereinstimmung: Ebenso wie in D1.

### Dokument D4: WO 98/35667 (TURNER NICHOLAS CHARLES ET AL.)

Es handelt sich um die Anwendung von NO-Synthasehemmem wie Aminoguanidin zur Behandlung des Typ II Diabetes.
Keinerlei Überschneidungen mit der hier eingereichten Patentanmeldung.

### Dokument D5: XP - 002276743 (JEFFREY C. STEVENS ET AL.)

Es handelt sich um die Substanz RG 12525, die als Xenobiotikum durch die Enzyme UGT1A1 und UGT1A3 glucuronidiert wird.
Keinerlei Überschneidungen mit der hier eingereichten Patentanmeldung.

Die Genese des Diabetes mellitus Typ II ist derzeit allenfalls in Teilschritten geklärt. Hier wird für eine Teilmenge des nicht-autoimmunopathischen und nicht-adipösen Diabetes ein völlig neues pathogenetisches Kausalkonzept postuliert, das dann konsequenterweise die Medikamentenzusammenstellung vorgibt. Es ist beabsichtigt, die von den Primärursachen ausgehende Krankheitsentwicklung an beliebiger Stelle des pathogenetischen Ablaufs so zu beeinflussen, daß sich der betroffene pathophysiologische Zustand bessert und höchstens noch eingeschränkt kausal fortpflanzen kann. Unabdingbar ist dafür das bisher nicht verfügbare kausale Ablaufschemä.

### C) Darstellung der Lösung und Beispiel

### 1. Ursachen

Es kann als gesichert gelten, daß die Entwicklung eines Diabetes mellitus II durch Gendefekte in die Wege geleitet wird, die Primärursachen oder wahrscheinlicher Dispositionen darstellen. Aufgrund der vielfältigen Ausprägungen müssen zahlreiche unterschiedliche Ursachen oder Dispositionen existieren. Weiterhin gilt als akzeptiert, daß bestimmten Verlaufsformen ein kybernetischer Hintergrund zukommt, d.h. daß ein konkret fehlgeleiteter Regelkreis der Glucosehomöostase zugrunde liegt.
Der L-Diabetes¹⁾ wird definiert als Teil des nicht-autoimmunopathischen und - nach manifester Erkrankung - nicht-adipösen Diabetes mellitus, der charakterisiert ist durch einen zentralnervös falsch niedrig gemessenen Ist-Wert der Blutglucose. Diese Falschmessung entsteht im ZNS durch die Abnahme der intra-/ extrazellulären ATP-Konzentration, die nicht durch eine Abnahme der Blutglucose verursacht, aber als solche interpretiert wird. Als Ursachen, die über eine kausale Verkettung die Minderung der cerebralen ATP-Bereitstellung bewirken, werden Gendefekte an Enzymen der UDP-Glucuronosyltransferase 1 (UGT1), eine genetische oder erworbene Überempfindlichkeit des sympathisches Systems, eine genetisch bedingte oder erworbene Unterversorgung mit Immunglobulin M (IgM) und Reaktionen auf unverträgliche Nahrungsmittel postuliert. Es ist nicht entschieden, in welcher Kombination und Ausprägung die ursächlichen Faktoren vorliegen müssen; weitere, die ATP-Erzeugung mindernde Einflußgrößen werden als Ursachen nicht ausgeschlossen.
***Hypothetisch wird angenommen, daß die Versorgung des Gehirns mit Glucose durch ATP-sensorische Zentren gemessen wird, so daß sich bei einer Abnahme der intra-*/ *extrazellulären ATP-Konzentration des ZNS die Interpretation "Hypoglykämie" bildet***.
Trifft diese Annahme zu, dann ist mit der Aufdeckung von Pathomechanismen, die cerebrale ATP-Bereitstellungen mindern, der Verlauf des L-Diabetes identifiziert.
¹⁾ Bezeichnung als **L**-Diabetes nach dem Patentanmelder **L**iebel.

### a) UGT1-Polymorphismen

Zur Familie der aktiven UGT1-Enzyme werden nach derzeitigen Erkenntnissen im einzelnen UGT1A1, UGT1A3, UGT1A4, UGT1A6, UGT1A7, UGT1A8, UGT1A9 und UGT1A10 gezählt. Diese Enzyme konjugieren Tausende von mehr oder weniger toxischen Substanzen. UGT1A1 glucuronidiert bevorzugt Bilirubin; die Glucuronidierungen der anderen Toxine, vor allem die Entgiftung der einem besonderen Augenmerk unterliegenden einfachen und komplexen Phenole, sind in der nachfolgenden Tabelle angegeben (Quelle: [3]).

| UGT1-Enzym | Phenole, einfach | Phenole, komplex | Amine, primär | Anthrachinone | Cumarine | Bilirubin |
|---|---|---|---|---|---|---|
| 1A1 | 1900 | 420 | 0 | 1720 | 800 | 400 |
| 1A3 | 239 | 299 | 84 | 1072 | 1970 | 0 |
| 1A4 | 30 | 11 | 540 | 0 | 0 | 2 |
| 1A6 | 2400 | 13300 | 10600 | 0 | 1100 | 0 |
| 1A7 | 175 | 480 | 0 | 57 | 220 | 0 |
| 1A8 | 1346 | 2217 | 42 | 1534 | 4970 | ND |
| 1A9 | 5300 | 1200 | 1800 | 2500 | 1500 | 0 |
| 1A10 | 88 | 85 | 0 | 35 | 11 | ND |
| Tab.1: UGT 1-Glucuronidierungsaktivität für ausgewählte Substanzklassen. | | | | | | |

*Angegeben sind maximale spezifische Aktivitäten (in picomol pro min und mg Protein), wobei für jede Substanzgruppe eine definierende Substanz verwendet wurde.*

Nichtglucuronidierte und damit verzögert entsorgte Phenole, z.B. die Benzo[a]pyren-Phenole aus der Menge der ubiqitär vorhandenen Polycyclischen Aromatischen Kohlenwasserstoffe (PAK), sind Präkursoren des radikalebildenden Hydrochinoncyclus. Zu beachten ist weiter, daß die UGT1-Familie große Anteile der Phase II-Konjugationen übernimmt, so daß bei einem Mangel an Glucuronidierungskapazität vermehrt schwefelverbrauchende Konjugationen eingesetzt werden müssen, mit der Gefahr einer Glutathiondepletion.

Erhöhte Aufmerksamkeit kommt dem Defektallel UGT1A1*28 zu, dessen Vorliegen eine verminderte Bilirubinglucuronidierung bedeutet. Fast immer treten zusammen mit UGT1A1*28 die Allele UGT1A6*2 und UGT1A7*3 auf, die dann Ursache einer defizitären Amin- und Phenol-Glucuronidierung sind. Deshalb steht für eine molekulargenetische Untersuchung der UGT1-Funktionalität, initiiert durch eine intermittierende Hyperbilirubinämie, zunächst die Suche nach UGT1A1*28 im Vordergrund. Trifft UGT1A1*28 zu, dann ist bei kaukasischen Bevölkerungsgruppen fast sicher mit UGT1A6*2 und UGT1A7*3 zu rechnen, deren Zutreffen durch eine Nachmessung i.a. bestätigt werden wird (vergl. [1]).
Bei *homozygotem* Vorliegen des Allels UGT1A1*28 kann ein Absinken der Enzymleistung auf 30 % - 60 % der Normalaktivität gegeben sein, bei *heterozygotem* Auftreten entsteht ein klinisches Bild i.a. nur im Fall extremer Belastung.

Die UGT-Enzymfamilien sind in Leber und Niere sowie gastrointestinal im Ösophagus, Magen, Duodenum, Jejunum, Ileum und Colon lokalisiert, eine geringe Aktivität ***liegt auch im Gehirn vor.*** Es besteht eine unterschiedliche, vom jeweiligen Enzym und vom betrachteten Gewebe abhängige Genexpression; die Enzyme UGT1A1 und UGT1A6 zeichnen sich dabei besonders aus, vergl. [3]: "The appearance of UGT1A1, UGT1A6, UGT2B4 and UGT2B7 mRNA showed the most dramatic variability between the different intestinal samples."
Vergl. [2]: "For example, in human stomach, the polymorphic expression of the UGT1A isoforms UGT1A1, UGT1A3 and UGT1A6 has been demonstrated. The variations correlate with a fourfold interindividual variation of glucuronidation activities for a number of phenolic compounds between individuals."

Aus diesem Grund ist bereits bei intakter UGT-Genausstattung von Person zu Person und von Gewebe zu Gewebe eine unterschiedliche Expression der zur UGT-Familie gehörenden Enzyme zu erwarten. Hinzu kommen dann die variierenden Schädigungen einzelner Enzyme durch untüchtige Allele, die zu einer langen Liste möglicher Defekte gehören, und die hetero- oder homozygot auftreten. Deshalb kann sich die Situation ergeben, daß bestimmte Personengruppen in bestimmten Geweben wegen spezifischer aktivitätsgeminderter UGT1-Enzyme eine dramatisch hohe Konzentration von einfachen und komplexen Phenolen zu gewärtigen haben, bei einem gleichzeitig normalen oder nur gering erhöhten Bilirubin.
Eine normale oder nur wenig erhöhte Bilirubinkonzentration ist durchaus auch für die Konstellation UGT1A1*28/*28 zu erwarten, da dieser homozygote UGT1A1-Defekt die Enzymleistung zwar auf 30% - 60% herabsetzt, wobei aber für das normal konstituierte Enzym 25% der Aktivität genügen, um eine Bilirubinglucuronidierung innerhalb der Norm zu erreichen.
Nahrungsmangel, Streß und körperliche Anstrengung senken die UGT1A1-Aktivität und erhöhen so Bilirubin. Ein bereits funktionsgemindertes UGT1A1-Enzym reagiert bei Streßbelastung mit deutlicherer Arbeitsverweigerung; es beläßt vermehrt unkonjugiertes Bilirubin in der Zirkulation und kann deshalb als eine *Feineinstellung der cytoprotektiven Bilirubinwirkung* angesehen werden (vergl.[1]).
Außer der Cytoprotektion durch das System Bilirubin/ Biliverdinreduktase kann bei einem UGT1-Defizit ein weiterer, evolutionär bedeutsamer Einfluß bestehen. Betrachtet man als ein Beispiel *Isoniazid,* so besteht der Wirkmechanismus dieser Substanz darin, daß die NAD-Blockierung und die durch Flavinenzyme kompensatorisch gesteigerte Oxidation des Substratwasserstoffs zu H₂O₂ verantwortlich sind für die Entstehung *reaktiver Sauerstoffmetabolite,* die wiederum die DNA, die Enzyme und die zellulären Membranen angreifen. Dadurch werden vor allem die stark proliferierenden Mykobakterien geschädigt.
Solche Sauerstoffradikale sind, um ein weiteres Beispiel zu nennen, auch das Wirkprinzip des gegen Blasenentzündungen verwendeten Hydrochinons *Arbutin.*
Analog zu den Beispielen Isoniazid und Arbutin können bei UGT1-Mangel die unzureichend glucuronidierten Superoxidradikalanionen-erzeugenden Substanzen, etwa die der Substanzklasse der polycyclischen aromatischen Kohlenwasserstoffe zugehörigen Chinone, gegen spezifische bakterielle Angriffe eine wirksame Abwehr bilden. Folgert man weiter, so wären die durch Bakterien verursachten Schäden, zu denen u.U. auch Gefäßschädigungen zählen, im langfristig gemessenen Umfang *verringert.*

### b) Überempfindlichkeit des sympathischen Nervensystems

Bei einem empfindlich reagierenden sympathischen System (ESS) können genetische und/ oder erworbene Ursachen vorliegen. Geht man von einem genetischen Hintergrund aus, so kann angenommen werden, daß psychosozialer Streß überdurchschnittlich schnell eine intensive und lang anhaltende Streßantwort auslöst. Daraus folgen extracerebral erhebliche Veränderungen, aber auch die an der Situationsbewältigung beteiligten Hirnareale sind in ihren Aktivitäten exorbitant gefordert und verbrauchen entsprechend viel ATP. Das Absinken der ATP-Konzentration als Folge von Denkprozessen ist eine physiologische Situation, die jedoch durch eine verringerte Befähigung zur ATP-Erzeugung so verschärft werden kann, daß für die Streßreaktion ein exponentieller Anstieg wahrscheinlich ist.
Aber auch ohne die Annahme genetischer Ursachen läßt sich eine Überreaktion auf Streß begründen:
- Ein bei Streßeinflüssen stets überhöhter zentraler Sympathikotonus kann dadurch gegeben sein, daß frühkindliche Streßerfahrung eine derartige Überempfindlichkeit bewirkt hat. So stellt die Flaschenfütterung für Neugeborene eine ganz außerordentlich hohe Streßbelastung dar, die zur generellen Etablierung einer lebenslang anhaltenden überhöhten Streßbeantwortung führen könnte.
- Die im ZNS gebildete Interpretation "Hypoglykämie" ist der *Stressor par excellence* und zweifellos befähigt, selbst bei geringer Intensität einer echten oder falschen Hypoglykämiemeldung eine energische Zunahme der Streßhormoneherbeizuführen

### c) Selektives IgM-Defizit Nahrungsmittelintoleranzen

Eine an der unteren Normgrenze gemessene IgM-Konzentration kann genetisch determiniert oder erworben sein, im letzteren Fall z.B. durch knochenmarkschädigende Wirkungen von Toxinen oder ionisierender Strahlung. Begleitend ist eine Tendenz zu erniedrigter Phagocytoseaktivität der Granulocyten festzustellen.

Die vornehmlich intravaskulär erscheinenden Immunglobuline M halten sich auch in der Lamina propria mucosae auf, wobei für die Konzentration [IgM] < [IgA] gilt. Bei einer dauerhaft unzureichenden Intensität der initialen, systemischen, IgM-vermittelten Immunantwort ist bei bakteriellem Angriff eine Schädigung der Enterozyten die logische Folge. Als Ursachen dieser Mukosaschädigung werden bakterielle Proteasen, freie Gallensäuren, hydroxylierte Fettsäuren, Alkohole oder Säuren diskutiert. Durch den an den Enterozyten festzustellenden Verlust der strukturellen Integrität sowie durch veränderte Enzymaktivitäten, gestörte Transportmechanismen und verminderte Permeabilitätsbarrieren entsteht eine entzündlich-atrophische Dünndarmschleimhaut, die zahlreichen Funktionseinschränkungen unterliegt.

Deutlichen Einfluß auf das intestinale Geschehen ist unverträglichen Nahrungsmitteln zuzuschreiben. Im allgemeinen ist der L-Diabetes *nicht* durch *IgE-Antikörper* gegen spezifische Lebensmittelbestandteile charakterisiert. Vielmehr handelt es sich um Nahrungsmittelintoleranzen, die keinen IgE-vermittelten Mechanismus besitzen. Ob und welche immunologischen Grundlagen zutreffen ist unbekannt; eine weitgehende Mitbeteiligung des Dünndarms anzunehmen ist jedoch naheliegend.

### d) Radikale

Als Folgeereignisse, entstanden aus den Ursachen a) bis c), besitzen Radikale weiterleitende oder übertragende Funktion; die vielfältigen und unübersichtlichen Möglichkeiten zur Radikalenbildung begründen jedoch auch eine Einstufung der Radikale als unabhängige Ursachen.
Der Mangel an Phenolkörper-glucuronidierenden UGT1-Enzymen bewirkt, daß die nichtentsorgten Phenolkörper über den Hydrochinoncyclus vermehrt Superoxidradikalanionen °O₂⁻ erzeugen, die durch Reaktionen vom Fenton-Typ zu Hydroxylradikalen °OH wie folgt umgesetzt werden:

°O₂⁻ + °O₂⁻ + 2H⁺→ H₂O₂ + O_{2.}

Fe³⁺ + °O₂⁻ → Fe²⁺ + O₂.

Fe²⁺ + H₂O₂ → Fe3⁺ + °OH + OH⁻ . (Fenton-Typ)

Bei Gegenwart von Sauerstoff werden die besonders aggressiven Hydoxylradikale °OH sowie Lipidperoxylradikale LOO° durch *ionisierende Strahlung* auch unmittelbar gebildet, die Hydoxylradikale °OH sogar *aus purem Wasser* (vergl. [4]):

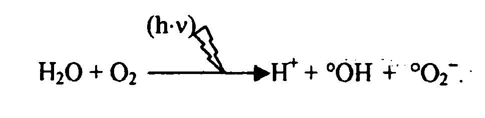

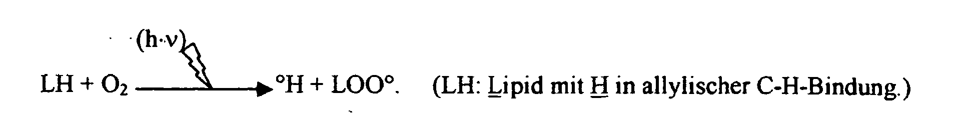

Radikale wirken zerstörend auf Membranlipide, auf SH-Gruppen in Aminosäuren sowie auf Thymin und Guanin in Nukleinsäuren. Die Peroxidation von Membranphospholipiden beginnt mit der Abstraktion eines Wasserstoffatoms aus der allylischen C-H-Bindung durch die Einwirkung von Radikalen und anderen reaktiven Initiatoren, insbesondere auch unmittelbar durch die Einwirkung *ionisierender Strahlung.* Eine Kettenreaktion wird in Gang gesetzt, die zu Lipidhydroperoxiden und immer neuen Lipidalkylradikalen führt. Durch Wechselwirkungen zwischen benachbarten Phospholipidmolekülen und im Rahmen von Terminationsvorgängen kommt es zur Bildung von Lipid-Lipid-, Lipid-Protein- und Protein-Protein-Vernetzungen (s. [4], S. 102). Die Folge ist eine Funktionsbeeinträchtigung der membrangebundenen Proteine und der Membranen insgesamt. Schäden an der Myoinositol-Signaltransduktion, den Ionentransportern, den Elektronentransportmolekülen der mitochondrialen Atmungskette und den Arachidonsäure-Abkömmlingen mit ihren Einflüssen auf das Kallikrein-Kinin-Prostaglandin-System sind zwangsläufig. ***Besonders betroffen sind Nerven- und Muskelzellen, die sich nicht mehr teilen. Die Erythrocyten verlieren ihre Formbarkeit und thrombosieren Kapillargefäße.***

Geht man von Gendefekten als einem disponierenden Hintergrund aus (eben diese Konstellation wird als Basis zugrunde gelegt), so bedarf es für die Manifestation des L-Diabetes eines auslösenden Ereignisses, z.B. einer lang andauernden Belastung durch psychosozialen Streß, durch exogene und endogene Schadstoffe, durch Toxine aus Depotfett bei Hungerkuren oder durch ionisierende Strahlung geringer Intensität aber langer Dauer. Bestehen schließlich - wie es sich beim pathogenetischen Konzept des L-Diabetes zeigt - zahlreiche positive Rückkopplungen (s. Abb. 2), so kann sich der Organismus bei wachsenden Schäden nicht mehr aus dem Teufelskreis befreien. Die sich bildenden pathophysiologischen Zustände werden positiv rückgekoppelt und damit gehalten und verstärkt, hinführend zu einem "point of no return".

### 2. Pathogenese

Die bakterielle Überbesiedlung des Dünndarms entsteht durch verminderte Darmmotilität, reduzierte intestinale Drüsensekretion, veränderte Enzymaktivitäten, erhöhte Durchlässigkeit der Perineabilitätsbarrieren und Reaktionen auf unverträgliche Nahrungsstoffe.
Aus Lysin, Arginin, Tyrosin, Ornithin, Histidin und Tryptophan ergeben sich durch Decarboxylierung die toxischen Amine Cadaverin, Agmatin, Tyramin, Putrescin, Histamin und Tryptamin. Außerdem wird Tryptophan in einer mehrstufigen Reaktion zu Indol und Scatol (Methylindol) umgewandelt, die nach partieller Resorption und Transport zur Leber mittels Sulfatkonjugation entgiftet werden müssen.
Des weiteren entstehen durch bakteriellen Angriff aus der Aminosäure Methionin die *extrem* toxischen Mercaptane wie Methanthiol, Ethanthiol, Dimethylsulfid und Dimethyldisulfid. Aus Phenylalanin und Tyrosin bilden sich toxische Phenole und Phenolderivate, deren Metabolisierung in der Leber u.a. p-Hydroxyphenylacetat und p-Hydroxyphenyllactat ergeben, die durch Veresterung mit Glucuronsäure oder Schwefelsäure zu detoxifizieren sind.
Als Folge des UGT1-Mangels müssen diejenigen endogenen und exogenen Giftstoffe, die hauptsächlich einer Glucuronidierung unterliegen, mit Hilfe anderer Konjugationen in eine ausscheidungsfähige Form gebracht werden. Hierzu dienen die Sulfat-, Glutathion- und Taurinkonjugationen mit der unvorteilhaften Nebenwirkung einer *starken Beanspruchung des Sulfat- und Glutathionpools.* Während UDP-Glucuronsäure nahezu unbegrenzt - allerdings unter Energieverbrauch - produziert werden kann, sind die nur in begrenzter Menge verfügbaren Aminosäuren Methionin und Cystein die Lieferanten der benötigten Schwefelverbindungen. Es zeigt sich eine meßbare Verminderung des reduzierten Glutathions, eine Erhöhung der Glutathionreduktase sowie eine Konzentrationserhöhung jener Toxine, die einer Konjugation mit Schwefelverbindungen bedürfen.
Am Beispiel der *in der Nahrung reichlich* enthaltenen Phenolcarbonsäuren und ihrer Ester werden die Auswirkungen der Konzentrationszunahmen von Phenolen gezeigt. Zu den Phenolcarbonsäuren gehören u.a. Hydroxyzimtsäuren (z.B. o-Cumarsäure, p-Cumarsäure, Kaffeesäure) sowie Hydroxybenzoesäuren (z.B. Salicylsäure, p-Hydroxybenzoesäure, Protocatechusäure). Ein Ester der Kaffeesäure ist die in vielen Pflanzen enthaltene Chlorogensäure.
Aus Hydroxyzimtsäuren können durch Abbau der Seitenkette über den Weg der ß-Oxidation die Hydroxybenzoesäuren entstehen, die wiederum durch Decarboxylierung einfache Phenole ergeben. Für die metabolische Aktivierung der Phenole gilt: Phenol → Hydrochinon → (schrittweise Oxidation) p-Benzochinon. Das p-Benzochinon ist Startsubstanz eines °O₂⁻ -erzeugenden *Cyclus:*

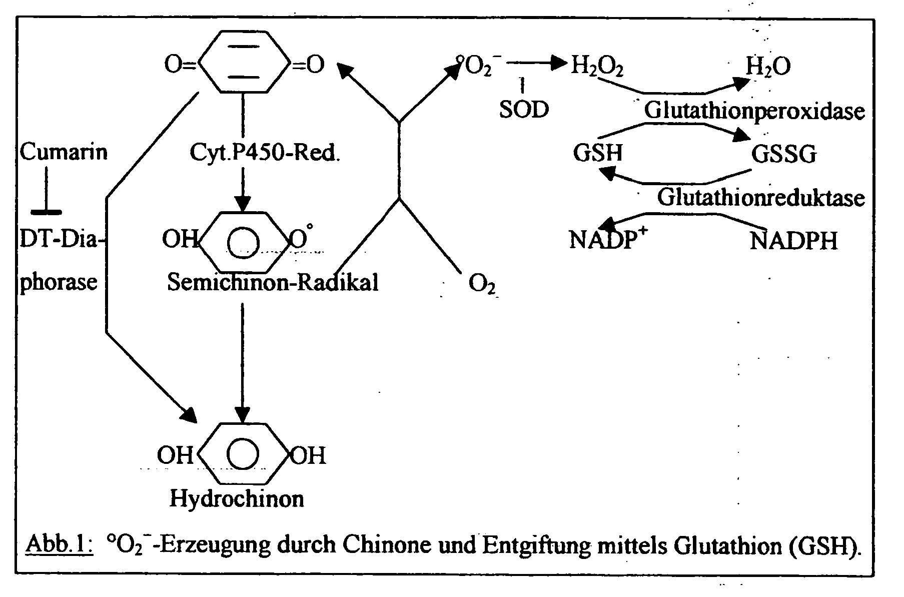

Die Abb.1 zeigt die Erzeugung des Superoxidradikalanions °O₂⁻ am *Beispiel des Hydrochinons.* Das Prinzip der dargestellten Radikalenerzeugung gilt ebenso für weitere Chinone, die etwa durch Oxidation polycyclischer aromatischer Kohlenwasserstoffe (PAK) entstehen. Wesentliche Erzeuger von °O₂⁻ sind die *Benzo[α]pyren-Phenole,* die wegen des Defektallels UGT1A7*3 vermutlich auch in hoher Konzentration vorhanden sind, denn es gilt (vergl. [1]): "UGT1A7*3 combines the allelic variation present in UGT1A7*2 and *4 and leads to the stongest reduction of enzyme activity towards benzo[a]pyrene phenols".
Das detaillierte Schema der *Pathogenese des L-Diabetes* zeigt Abb.2:

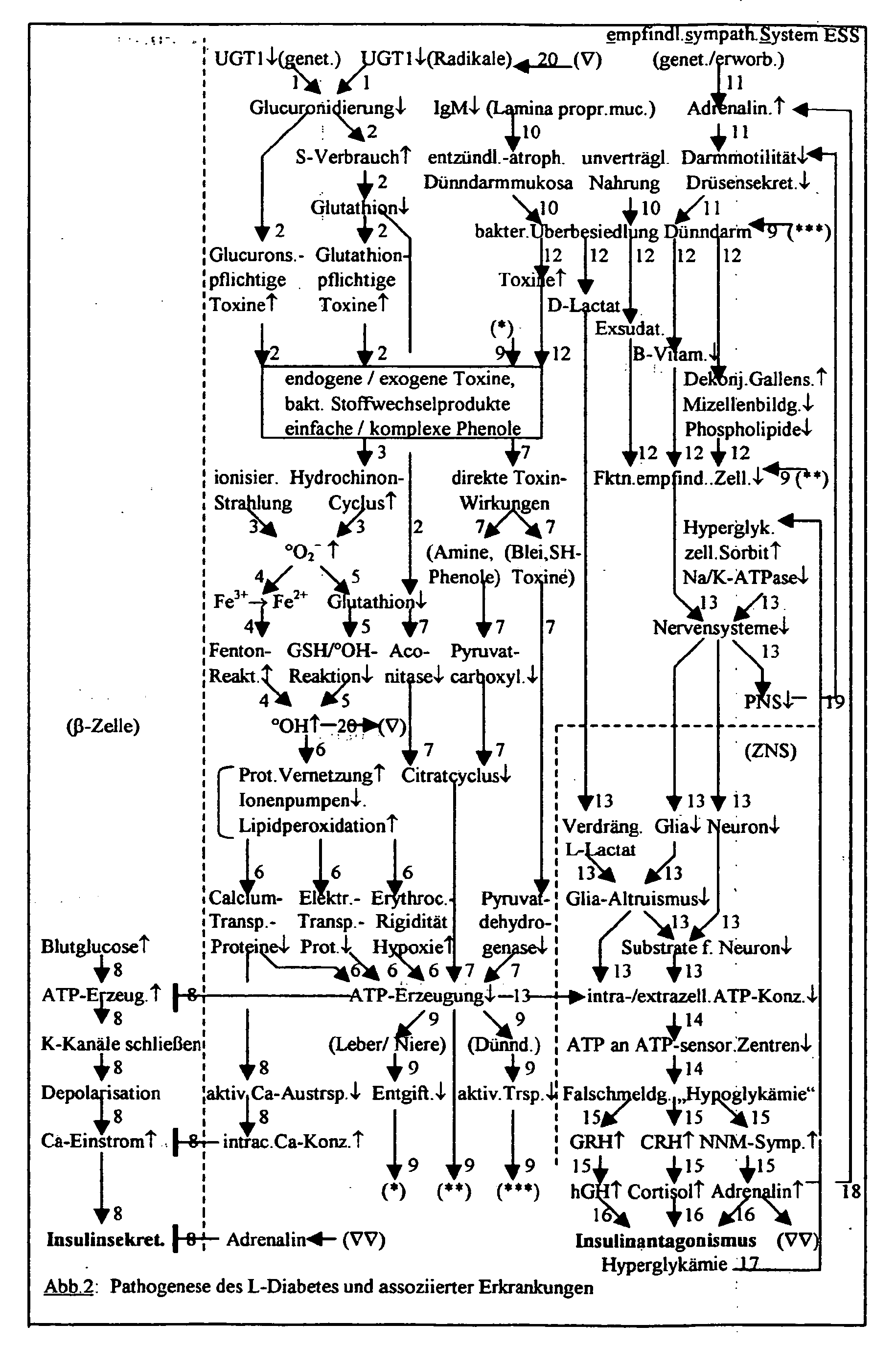

Die im Ablaufschema der Abb.2 enthaltenen Übergänge bedeuten im einzelnen:
1 Ausgehend von dem Vorliegen der Defektkonstellation [UGT1A1*28/*28, UGT1A6*2/*2, UGT1A7*3/*3], sowie unter Beachtung der insbesondere für die UDP-Glucuronosyltransferase sich auswirkenden Radikalenanhäufung (siehe [4], S. 102), kann ein Glucuronidierungsdefizit gefolgert werden.
2 Für nichtglucuronidierte Schadstoffe müssen vermehrt schwefelverbrauchende Konjugationen herangezogen werden, so daß der Glutathionbestand abnimmt.
3 Nicht oder nur verzögert entsorgte einfache und komplexe Phenole erzeugen über den Hydrochinoncyclus die Superoxidradikalanionen °O₂⁻. Auch andere Quellen sind beteiligt, von denen die ionisierende Strahlung als Beispiel dient.
4 Die Superoxidradikalanionen °O₂⁻ überführen n-wertige Übergangsmetallionen in (n-1)-wertige Metallionen, die dann mit H₂O₂ in einer Reaktion vom Fenton-Typ die besonders aggressiven Hydroxylradikale °OH bilden.
5 Glutathion interagiert mit den Superoxidradikalanionen °O₂⁻ und den Hydroxylradikalen °OH in konkurrierenden GSH-°O₂⁻ - bzw. GSH-°OH-Reaktionen (siehe [4], S. 104); die GSH-°O₂⁻-Reaktion verbraucht Glutathion und benachteiligt bei knappem Bestand die Beseitigung der Zellstrukturaggressoren °OH.
6 Die Einwirkungen von Radikalen führen zu Peroxidationen von Membranbestandteilen und zu Funktionsverlusten membrangebundener Proteine. Enzyme, ATP-abhängige Calciumtransportproteine und Elektronentransportproteine der mitochondrialen Atmungskette sind ebenso betroffen wie Bestandteile der Erythrocyten, die dadurch in ihrer Deformierbarkeit bei der Passage von Kapillargefäßen und ihrer Befähigung zum Sauerstofftransport nachlassen.
7 Den Citratcyclus definierende Enzyme und anaplerotische Enzyme sind durch Glutathionmangel und direkte Toxinwirkung gefährdet: Die *Aconitase* enthält Fe(II) und benötigt reduziertes Glutathion, die *Pyruvatcarboxylase* wird durch Urämietoxine wie Guanidine, Phenole und Amine gravierend beeinträchtigt. Die *Pyruvatdehydrogenase* und die *SH-Enzyme der Glycolyse* werden durch Sulfhydrylgruppen-toxische Substanzen inhibiert. Die ATP-Synthese nimmt ab.
8 Die physiologische Sekretion von Insulin bei Erhöhung der Blutglucose wird gehemmt, wenn die β-Zellen eine verminderte Befähigung zur ATP-Bildung entwickeln. Eine weitere Sekretionshemmung entsteht, falls defekte Calciumtransportproteine den aktiven Austransport von Calcium aus der β-Zelle nicht bewältigen und ein permanent überhöhter intracellulärer Calciumspiegel die Insulinspeicher erschöpft. Hemmenden Einfluß an der β-Zelle besitzt außerdem ein überhöhter Sympathikotonus: Das vermehrt auftretende Adrenalin setzt die Insulinsekretion über ∝₂-Adrenorezeptoren herab; der bei hohem Sympathikotonus gegenläufig abgesenkte Vagotonus (nicht in Abb.2 eingezeichnet) trägt zur Mangelsekretion bei, da eine modulierende Sekretionserhöhung mittels Acetylcholin und muskarinischer Rezeptoren ausfällt.
9 Die verminderte ATP-Erzeugung wird ***positiv rückgekoppelt:*** ATP-Mangel verringert die Entgiftung durch Leber und Niere (*), mindert die Funktion von Zellen mit hohem Stoffwechsel (**) und beeinträchtigt den aktiven Transport von Proteinen aus dem Darmlumen (***); dadurch werden die Toxinlast und nachfolgend die ATP-Synthesehemmung gehalten und verstärkt.
10 Durch eine genetisch bedingte oder erworbene Unterversorgung mit IgM und die Tendenz zu erniedrigter Phagocytosefähigkeit der Granulocyten entsteht eine entzündlich-atrophische Dünndarmmukosa, deren Funktionseinschränkungen bakterielle Überbesiedlungen mitverursachen. ***Unverträgliche Nahrungsmittel (im Verdacht: Milchprodukte, Milchpulver, Getreide) bewirken individuell unterschiedlich starke intestinale Reaktionen mit Beteiligung des Dünndarms.***
11 Das bei Streßeinwirkung empfindlich reagierende sympathische System erhöht die Adrenalinausschüttung und bewirkt auch unmittelbar über sympathische Fasern (in Abb.2 nicht eingezeichnet) eine Abnahme der Darmmotilität und der Sekretion intestinaler Drüsen; die Bakterienansiedlung im Ileum nimmt zu.
12 Die unphysiologische bakterielle Überbesiedlung des Dünndarms erzeugt Toxine, unter anderem D-Lactat. Es entstehen Verluste durch Exsudation und durch die Entnahme von Vitaminen und Spurenelementen aus dem Chymus für den bakteriellen Stoffwechsel. Die bakteriell dekonjugierten Gallensäuren hindern die Mizellenbildung und die Aufnahme fettlöslicher Vitamine und Phospholipide. Für Zellen mit hohem Stoffwechsel oder Bedarf an spezifischen Substraten, z.B. Phospholipiden für Myelinscheiden, entstehen Funktioriseinbußen.
13 Die generelle Abnahme der ATP-Erzeugung führt auch im Zentralnervensystem zu einer entsprechenden Mangelsituation. Erschwerend kommt hinzu, daß die Neuronen des ZNS auf eine Versorgung mit L-Lactat durch Gliazellen angewiesen sind, die bei hohen D-Lactatspiegeln, erzeugt durch intestinale bakterielle Fehlbesiedlung, gestört ist. Die Glia-Zellen weisen zudem *hohe Aktivitäten der Enzyme des Polyolwegs* auf, so daß sich bei diabetischer Hyperglykämie vermehrt Sorbit bildet, das die Zelle nicht mehr verlassen kann und eine hyperosmolare Schädigung bewirkt. Die als Folge hoher Glucosekonzentrationen verringerte zelluläre Aufnahme von Inositol mit herabgesetzter Bildung von Phosphatidylinositol-4,5-Bisphosphat und nachlassender Aktivierung der Proteinkinase C resultiert in einer Aktivitätsabnahme der Na/K-ATPase.
14 Es wird hypothetisch gesetzt, daß ATP-sensorische Gehirnzentren niedrige ATP-Konzentrationen als Ist-Werte erfassen, mit den vorgegebenen SollWerten vergleichen und einer Stoffwechselsituation zuordnen, die "Energiemangel" lautet. Da *physiologischerweise* ein Energiemangel durch einen Mangel an Glucose entsteht, wird bei der Registrierung einer verminderte ATP-Konzentration ein Glucosedefizit als Ursache gedeutet und die Alarmmeldung "Hypoglykämie" ausgegeben.
15 Die Interpretation "Hypoglykämie", die im Fall einer *durch Radikale und Toxine bewirkten Abnahme der ATP-Erzeugung* eine Falschmeldung darstellt, führt konsequenterweise über die Achse CRH→ACTH→NNR zu Cortisol, über die Verbindung GRH→Hypophyse zu Wachstumshormon (hGH) und über den Nebennierenmark-Sympathikus (NNM-Sympathikus) zu Adrenalin. Nicht in Abb.2 eingezeichnet ist eine von den ATP-sensorischen Gehirnzentren ausgehende Absenkung des Vagotonus; die wegfallende Aktivierung der zum Pankreas führenden vagalen Fasern ergibt, daß eine ansonsten mögliche modulierende Sekretionsstimulation ausbleibt.
16 ***Trotz eventuell hoher Blutglucosewerte ist es von der Höhe des Defizits an zentralnervös benötigten Energiesubstraten abhängig, in welcher Intensität Glucoseproduktion und Insulinantagonismus weitergeführt werden.***
17 Positive Rückkopplung: Blutglucoseanstiege führen über die bewirkte Sorbiterhöhung und Na/K-ATPase-Verminderung zurück in den Pathogeneseweg.
18 Positive Rückkopplung: Erhöhung des Adrenalins führt über die Hemmung der Darmmotilität und Drüsensekretion zurück in den Pathogeneseweg.
19 Positive Rückkopplung: Schäden an peripheren Nerven, insbesondere Darmnerven, führen über die gehemmte Darmmotilität zurück in den Pathogeneseweg.
20 Positive Rückkopplung: Radikale↑ → UGT1↓ → Toxinlast ↑→ Radikale↑.

Trotz kausaler Zuordnungen zeigt die Abb.2 kein Kausalnetz, da kausale Netzwerke keine geschlossenen Kreisläufe aufweisen. Aus diesem Grund liegt auch kein sogenanntes L-Netz vor, das darüber hinaus ausschließlich pathophysiologische Zustände als Netzknoten zuläßt (siehe [5]). Dennoch gelten auch für das kausale Äblaufschema der Abb.2 wesentliche Strukturprinzipien der L-Netze:
Ein Übergang (*A* → *B*) besitzt Hemmungsmechanismen, die der Entstehung von *B* entgegenwirken und die Bildung von *B* unter Umständen zeitweise auch ganz vereiteln. Die Hemmungsmechanismen selbst sind ebenfalls hemmenden Einflüssen ausgesetzt, so daß auch akzeleriert erscheinende Übergänge erklärt sind (siehe hierzu [5], S. 10 - 17).
Die Gesamtheit aller Inhibitoren eines einzelnen Übergangs *(A → B)* ist im allgemeinen unbekannt. Das abstrahierte Ziel der Medikation ist es, die im Ablaufschema deklarierte Entwicklung eines beliebigen pathophysiologischen Zustands *B* aus dem kausal davor stehenden Zustand *A* zu hemmen oder aber die Hemmungsmechanismen des Übergangs dadurch zu aktivieren, daß deren Inhibition aufgehoben wird.
So sind die in Abb.2 implizit vorhandenen aber nicht eingezeichneten Inhibitoren beliebiger Vorgänge *(A → B)* mitverantwortlich für interindividuelle Abweichungen des Geschehens und zeitlich unterschiedliche Abfolgen.
Die in Abb. 2 aufgezeigten Rückkopplungen bewirken allmählich zunehmende, den ***Alterungsvorgängen*** vergleichbare Funktionseinbußen. Dies führt zu der Feststellung, daß primäre genetische Schäden nicht zwingend erforderlich zu sein scheinen, um die Pathogenese eines L-Diabetes zu starten. Die Erkrankung setzt aber früher und entschieden progressiver ein, wenn Gendefekte als Primärursachen vorliegen oder Mutationen im Lebensverlauf auftreten oder genetische Besonderheiten die Inhibitoren kausaler Übergänge verringern oder außergewöhnlich hohe chronische Belastungen die Inhibition der pathogenetischen Kausalverbindungen zu irgend einem Zeitpunkt herabsetzen.

Etliche Erkrankungen treten zusammen mit dem L-Diabetes oder auch isoliert auf:
- Cheiropathie,
- periphere Neuropathie,
- distale Muskelatrophie,
- extreme Muskelermüdbarkeit,
- beschleunigte cerebrale Ermüdbarkeit,
- Kataraktbildung,
- retinale Mikroaneurysmen,
- männliche Sexualfunktionsstörungen,
- Urämietoxinsymptomatik,
- Leberfunktionsstörungen und
- portale Hypertension.
Das Auftreten des L-Diabetes zusammen mit einzelnen der genannten Erkrankungen ist im allgemeinen die Folge *gemeinsamer Ursachen,* von denen die jeweiligen pathophysiologischen Entwicklungen ihren Ausgang nehmen. In der Liste sind jedoch auch Krankheiten aufgeführt, insbesondere die *Überlastung der Leber und der Nieren, die integraler Bestandteil* des L-diabetischen Kausalkonzepts sind.
Myotonie der Handmuskeln, periphere Muskelschwäche, Katarakte, Hodenatrophie und Diabetes mellitus sind Symptome der - mit 1:7500 allerdings seltenen - Myotonen Dystrophie, die durch eine molekulargenetische Untersuchung differentialdiagnostisch ausgeschlossen werden kann.
Der L-Diabetes führt im allgemeinen nicht zu Adipositas, da das lipogene Insulin supprimiert wird, und da zudem Hungergefühl und Nahrungsaufnahme durch Streßhormone zurückgedrängt werden. Ein nach klassischem Glucosebelastungstest einsetzender Hyperinsulinismus gehört *nicht* zu den Symptomen des L-Diabetes (bereits prädiabetisch zeigt sich ein Zuwenig an Insulineffekt anhand der Glucosewerte des Tests, die nach einer Stunde den Bereich 250 mg/dl erreichen).

Beispiele *exogener* Toxine sind in der nachfolgenden Tabelle genannt. Für die Entgiftung der Substanzen 1) - 10) werden hauptsächlich Glucuronidierungen genutzt, *bei UGT1-Defekten zunehmend Sulfatierungen,* so daß für die schwefelverbrauchende Entgiftung der Stoffe 11) - 21) nur wenig Schwefel zur Verfügung steht (Lit.: [4]).

**Tab.2: Exemplarische Auswahl exogener Toxine.**

| Toxin | | Zufuhr durch: | Hauptsächliche Entgiftung: |
|---|---|---|---|
| 1) | Polycyclische aromatische Kohlenwasserstoffe (PAK) | Pyrolyseprodukte, kontaminierte oberirdisch wachsende Pflanzen | Glucuronidierung, Sulfatierung, Glutathionkopplung |
| 2) | N-Nitrosoverbindungen | Nitratgedüngte Gemüse | Glucuronidierung |
| 3) | Benzol / Phenol | Atemluft, Lebensmittel, Trinkwasser | Glucuronidierung, Sulfatierung, Glutathionkopplung |
| 4) | Aromatische Amine | Verunreinigte Umwelt | Glucuronidierung, Sulfatierung, |
| 5) | Heterocycl. aromat. Amine | Pyrolyseprodukte | Glucuronidierung |
| 6) | Solanine | Nachtschattengewächse | Glucuronidierung |
| 7) | Dibenzodioxine, Dibenzofurane | Milch, Fleisch, Eier | Nach Hydroxylierung entgiftet durch Glucuronidierung |
| 8) | Pentachlorphenol (PCP) | Verunreinigte Nahrung | Glucuronidierung |
| 9) | Chlorogensäure, Kaffeesäure, Protocatechusäure | Möhren, Sellerie, Kohl, Kartoffel, Kaffee, Schwarztee, Kakao, Wein, Kopfsalat, Endivie | Nach der Metabolisierung zu einfachen Phenolen erfolgt Glucuronidierung |
| 10) | Cumarine | Getreide, Hülsenfrüchte. Möhren. Fenchel, Nachtschatten, Citrus, | Hydroxylierung, dann Glucuronidierung und andere Konjugat |
| 11) | Acrylamid | Brot, Gebäck, Kaffee, Kakao, Bratkartoffel, Kartoffelchips | Glutathionkopplung |
| 12) | Polychl. Biphenyle (PCB) | Milch, Fleisch, Fisch | Glutathionkopplung |
| 13) | Arsen | Inhalative Aufnahme | Glutathion antagonisiert |
| 14) | Quecksilber | Frischwasser- und Seefische | Glutathion bindet Hg-Salze |
| 15) | Cadmium | Ölsaaten, Spinat, Schokoladen | Cystein (Metallothionein) |
| 16) | Blei | Bleibenzin, Milch, Vit. D-Mangel | Cystein schützt δ-ALA-Dehydr. |
| 17) | DDT | Verunreinigte Nahrung | Glutathion-abh. Abbau zu DDE |
| 18) | Organophosphate | Verunreinigte Nahrung | Glutathion-abh. CH₃- Transfer |
| 19) | Patulin | Mehl, Fleisch, braunfaule Äpfel | Hohe Affinität zu Cystein-SH |
| 20) | Penicillinsäure | Lebensmittel, Tierfutter | Führt zu Glutathiondepletion |
| 21) | Trichothecene | Getreide, Tierfutter | Hohe Affinität zu Cystein-SH |

### 3. Diagnostik, Prophylaxe, Therapie

Günstig ist eine frühzeitige Diagnose anhand der Ursachen oder der Frühsymptome. Die Homozygotie für das Allel UGT1A1*28 verrät sich durch eine leichte Hyperbilirubinämie, die etwa den 2- bis 3-fachen Normwert erreicht. Ist dieses Symptom gegeben, so ist eine molekulargenetische Untersuchung angebracht. Ergibt der Test ein Vorliegen von UGT1A1*28, so ist bei Kaukasiem fast sicher mit den ebenfalls leistungsmindernden Allelen UGT1 A6*2 und UGT1 A7*3 zu rechnen. Die Messung weiterer UGT1-Isoenzyme vervollständigt die Kenntnis über die vorhandene Glucuronidierungskapazität.
Wird die Hyperbilirubinämie begleitet von einer Verringerung des reduzierten Glutathions und einer Erhöhung der Glutathionreduktase, so sind dies deutliche Anzeichen einer unzureichenden Befähigung zur Glucuronidierung, so daß kompensatorisch der Anteil an schwefelverbrauchenden Konjugationen stark erhöht sein kann. Für diese Symptomatik scheint die Defektkonstellation [UGT1A1*28/*28, UGT1A6*2/*2, UGT1A7*3/*3] bereits ausreichend zu sein.
Die Messung der Immunglobulinkonzentration i.S. ist Routine. Hyperkortisolismus und Wachstumshormonüberschuß können durch Messung der Plasmakonzentrationen über einen 24h-Zeitraum bestätigt werden. Eine Adrenalinüberhöhung bei Streßsituationen ist anhand der mit dem Urin ausgeschiedenen Metaboliten erkennbar, allerdings ist die *Bewertung* der Catecholaminmessungen von Veränderungen der ebenfalls zu bestimmenden Schilddrüsenhormonspiegel stark beeinflußt.

Die Bestimmung der Ausscheidungsrate von Noradrenalin (NA) und Adrenalin (A) ist eine Methode zur Identifizierung eines empfindlichen sympathische Systems (ESS). Es zeigt sich, daß Probanden mit ESS im Verlauf einer 24h-Periode NA/A-Quotienten aufweisen, die deutlich niedriger sind als bei gesunden Erwachsenen. Die Schaubilder der NA/A-Quotienten verdeutlichen, daß der Mittelwert bei ESS ca. die Hälfte des Wertes beträgt, den Gesunde erreichen. Zudem ist bei Abwesenheit überhoher Streßempfindlichkeit meistens zu beobachten, daß die NA/A-Kurven weniger abrupte Schwankungen aufweisen (siehe [6]). Als Beispiele dienen die Abb. 3 bis 5:

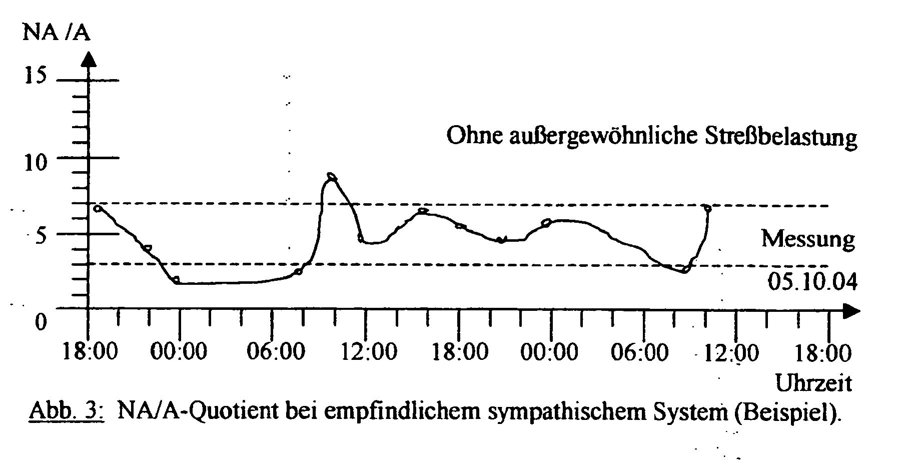

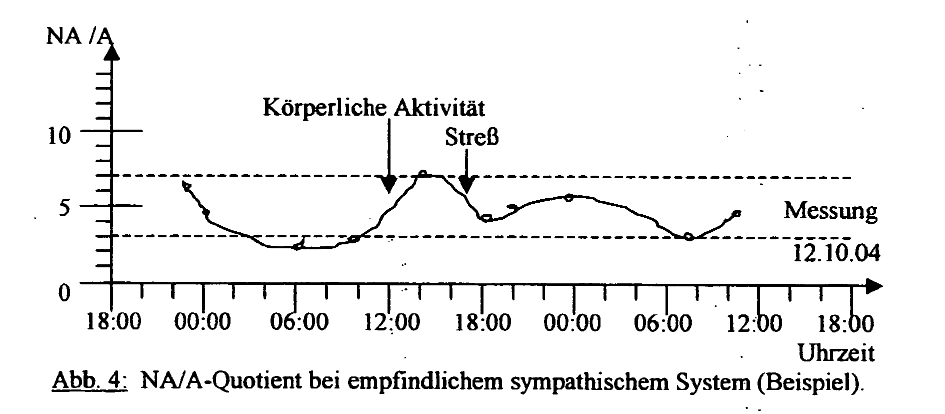

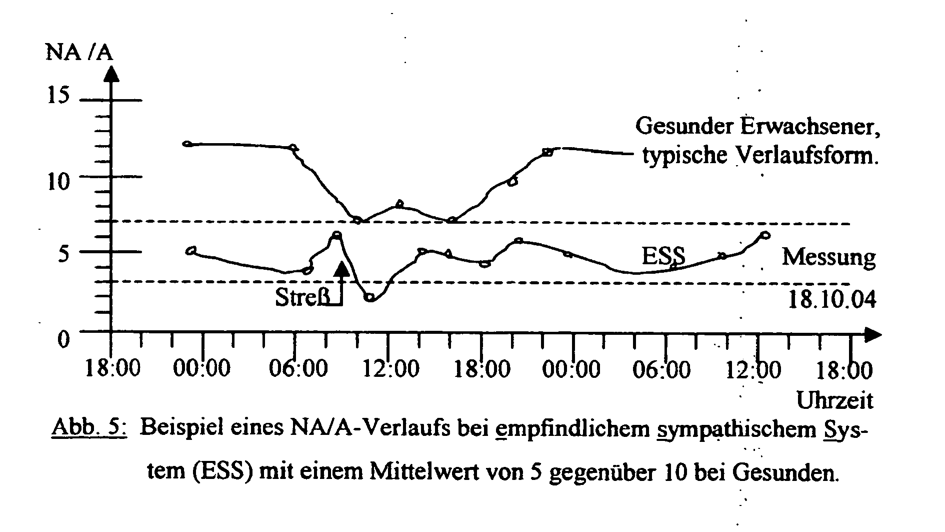

Der unteren Kurve in Abb. 5 liegt die folgende Meßwertetabelle zugrunde (im Fettdruck die Werte nach ½-stündiger Streßbelastung durch Zivilprozeß):

| Uhrzeit | 2345 | 0730 | 0915 | 1100 | 1440 | 1610 | 1830 | 2120 | 2350 | 0710 | 0940 | 1300 |
|---|---|---|---|---|---|---|---|---|---|---|---|---|
| NA [ng/min] | 32,7 | 18,9 | 56,6 | **98,6** | 21,9 | 37,2 | 37,8 | 42,0 | 28,9 | 16,6 | 45,9 | 40,2 |
| A [ng/min] | 6,2 | 4,9 | 8,9 | **39,7** | 4,5 | 7,7 | 8,5 | 6,9 | 5,7 | 4,0 | 10,4 | 6,2 |
| NA/A | 5,2 | 3,8 | 6,3 | **2,5** | 4,9 | 4,8 | 4,4 | 6,1 | 5,0 | 4,2 | 4,4 | 6,4 |

Der in den Abb. 3 bis 5 markierte Bezirk zwischen den NA/A-Werten 3 und 7 ist ein Indikator für die Bereitschaft und Befähigung zu maximaler Konzentration. Personen mit ESS weisen NA/A-Kurven in diesem Bereich auf, ebenso wie Hochleistungssportler vor und während der Absolvierung bedeutender Wettkämpfe (siehe [6]).

Anzuwenden ist eine Therapie, die an den Ursachen ansetzt, d.h. die der Entstehung von Radikalen und Toxinen entgegenwirkt, und die eine im ZNS entstehende Falschmeldung "Hypoglykämie" vermindert. Dazu muß zuvorderst die Blutglucose durch ***exogenes Insulin*** in der Nähe des Normwerts gehalten werden, u.a. um eine hyperosmolare, durch Sorbit verursachte Beeinträchtigung der Glia-Zellen zu vermeiden. Weiter müssen die Giftstoffe vermindert werden, z.B. die in nahezu allen *Pyrolyseprodukten* reichlich enthaltenen Substanzen des Hydrochinon-Typs sowie *alle Nahrungsmittel, die sich durch Unverträglichkeit auszeichnen.* Der Einsatz von Medikamenten mit ungünstigen Nebenwirkungen ist generell kontraindiziert, da eine langdauernde, unter Umständen lebenslange Zufuhr vorzusehen ist.

Die im pathogenetischen Kausalkonzept des L-Diabetes bedeutsame
1. Überbeanspruchung der schwefelverbrauchenden Phase II-Konjugationen,
2. die intensive Radikalenwirkung vor allem durch den verstärkt ablaufenden Hydrochinoncyclus und den Glutathionmangel,
3. die Resorptionsminderung von Phospholipiden sowie
4. die verminderte Aufnahme von fettlöslichen Vitamine und essentiellen mehrfach ungesättigten Fettsäuren infolge gestörter Mizellenbildung,
5. die Minderversorgung mit B-Vitaminen und Spurenelementen als Folge der bakteriellen Verstoffwechselung dieser Substanzen im Dünndarm und
6. die Schädigung der Dünndarmmukosa durch mangelnde IgM-vermittelte Immunabwehr bei bakteriellen Angriffen
werden durch eine *Kombination* von Arzneimitteln wie folgt therapiert:
- Zu 1.: Für die Behebung des Defizits an Schwefelverbindungen können N-Acetylcystein, L-Cystin, Glutathion, Taurin, L-Methionin und S-Adenosylmethionin verabreicht werden. Vorsicht ist jedoch geboten, da aus nicht-resorbiertem *Methionin* durch bakteriellen Abbau im Colon die *extrem* toxischen Mercaptane entstehen. Geeignet ist N-Acetylcystein, 3 x 200 mg / d.
- Zu 2.: Es können, um die Wirkungen der Radikale zu verringern, alle antioxidativ wirkenden Substanzen von Nutzen sein: Vitamin C, Vitamin E, α-Liponsäure, β-Carotin, Selen, Mangan, Rutosid, Hesperidin und Silymarin. *Vitamin E soll per Injektion verabreicht werden, um die bakterielle Überbesiedlung des Dünndarms nicht zu begünstigen.*
- Zu 3.: Durch bakteriell dekonjugierte oder hydroxylierte Gallensäuren wird die Mizellenbildung erschwert. Eine daraus resultierende Unterversorgung mit Phospholipiden (Lecithine, Kephaline, Phosphatidylinositol) und Phospholipidkomponenten (Cholin, Inositol) ist durch erhöhte Zufuhr zu beheben.
- Zu 4.: Die verminderte Resorption der fettlöslichen Vitaminen A und D sowie der essentiellen Omega-3- und Omega-6-Fettsäuren sollte durch Präparate oder entsprechende Nahrungszusammensetzung ausgeglichen werden. Als Cholagogum eignen sich Extrakte aus Rhizoma Curcumae xanthorrhizae.
- Zu 5.: Da die an der Überbesiedlung des Dünndarms beteiligten Bakterien die Aufnahme von B₁₂ im terminalen Ileum erschweren, und da sie auch für ihren eigenen Stoffwechsel Vitamine und Spurenelemente aus dem Chymus absorbieren, entstehen zu substituierendeVerluste an B-Vitaminen, Folsäure und Zink. *Die Zufuhr der B-Vitamine und der Folsäure erfolgt parenteral, da so das intestinale bakterielle Wachstum nicht gefördert wird.*
- Zu 6.: Die Therapie eines entzündlich-atrophischen Dünndarms ist zu versuchen; u.U. bietet das entzündungshemmende und epithelisierungsfördernde Dexpanthenol eine Möglichkeit.

Ein Stopp oder zumindest eine verlangsamte Entwicklung diabetischer Spätfolgen darf erwartet werden. Für eine restitutio ad integrum fehlen die Einwirkungsmöglichkeiten auf die Primärursachen.

Zur Therapie ungeeignet erscheinen derzeit die vielfach diskutierten *Methylxanthine:* Die Adenosin-empfindlichen P₁-Purinrezeptoren werden durch Methylxanthine geblockt, die ATP-akzeptierenden P₂-Purinrezeptoren bleiben unbeeinflußt. Damit steigt zentral das Verhältnis (P₂:P₁), d.h. das im ZNS registrierte Verhältnis der (ATP:Adenosin)-Konzentration nimmt zu. *Kurzfristig* ist jedoch kein bessernder Einfluß auf die Glucosehomöostase zu erkennen, weil entweder ein Anstieg des (P₂:P₁)-Wertes, herbeigeführt durch eine P₁-Verringerung, richtigerweise nicht als Verbesserung der cerebralen Energieversorgung interpretiert wird, oder weil Purinrezeptoren keine Meßfühler der ATP-sensorischen Gehirnzentren sind. Ethanol hemmt die hepatische Gluconeogenese als Folge der intensiven NADH-Produktion und der Überführung des Pyruvats in Lactat. Eine zu kappe ATP-Erzeugung im ZNS kann Ethanol nur dann verbessern, wenn der Defekt die Ebene der Pyruvatdehydrogenase, d.h. die zentralnervöse Bereitstellung von Acetyl-CoA betrifft: Das aus Ethanol hepatisch produzierte Acetyl-CoA wird bei einem Überschuß in der Leber zu β-Hydroxybutyrat umgesetzt, das nach der Blutpassage die Blut-Hirn-Schranke im Austausch gegen Lactat überwindet; bei der Verstoffwechselung im Gehirn ergibt β-Hydroxybutyrat erneut Acetyl-CoA und stützt so einen eventuell mit genau diesem Substrat unterversorgten Citratcyclus. Da jedoch der hepatisch anfallende Acetaldehyd an reduziertes Glutathion bindet und so die knappe Glutathionverfügbarkeit verschärft, sind allenfalls kleine Mengen Ethanol tolerierbar.

### Zitierte Quellen

[1] Köhle, C. et al.: Frequent co-occurence of the TATA box mutation associated with Gilbert's syndrome (UGT1A1*28) with other polymorphisms of the UDP-glucuronosyltransferase-1 locus (UGT1A6*2 and UGT1A7*3) in Caucasians and Egyptians. Biochemical Pharmacology 65 (2003), pp 1521 - 1527.
[2] Tukey, R.H. and Christian P. Strassburg: Human UDP-Glucuronosyltransferases: Metabolism, Expression, and Disease. Annual Reviews Pharmacology Toxicology 2000. 40: 581 - 616.
[3] Strassburg, C.P. et al.: Polymorphic Gene Regulation and Interindividual Variation of UDP-glucuronosyltransferase Activity in Human Small Intestine. The Journal of Biological Chemistry (2000), Vol. 275, No. 46, pp 36164 - 36171.
[4] Marquardt, H. und S. G. Schäfer (Hrsg.): Lehrbuch der Toxikologie. BI Wissenschaftsverlag (1994). ISBN 3-411-16321-6.
[5] Liebel, F.-P.: Berechnung kausaler Strukturen. Pabst Science Publishers Lengerich, Berlin, Bremen (2002). ISBN 3-89967-011-6.
[6] Zimmermann, E.: Leistung und Schlaf bei Sportlern. Wien. med. Wschr., Jg. 146, Heft 13/14 (1996) S. 280 - 282.

### Ergänzung zu

### B) Stand der Technik und Vorteile des Verfahrens

### Dokument D1*: EP-A2-0 768 043 BRISTOL-MYERS SOUIBB COMPANY)

Dokument 1* beschreibt Diabetikerriegel.

Das Ziel des Dokuments 1* ist es, eine kontrollierte oder fortwährende Absorption von Kohlenhydraten während der Verdauung zu erreichen. Dazu heißt es in der Description, Seite 2, Zeile 26: " The present invention is directed to ... ingredients that provide a fast, moderate and slow absorption of carbohydrate."
Hinzugefügt werden Vitamine, Mineralien und sonstige Stoffe, " ... that are known to be necessary for normal physiological function.". [Description, Seite 4, Zeile 11.]

### Dokument D2*: US 2003 / 134851 A1 BAXTER JEFFREY H ET AL)

Dokument D2* beschreibt ein flüssiges Präparat, das im wesentlichen N-Acetyl-L-Glutamin zuführen soll. In Claim 6 ist der Anwendungsbereich angegeben, das sind Magen-Darm-Erkrankungen oder allgemeiner Beeinträchtigungen des Verdauungskanals; Diabetes wird in den Claims nicht erwähnt.
Vitamine, Mineralien und sonstige Stoffe werden hinzugefügt, " ... to provide a more nutritionally complete and balanced formula" [Absatz 0070] bzw. " ... to supply the daily nutritional requirements" [Absatz 0071]. Auch steht in [Absatz 0071] der einzige Hinweis auf Diabetes: "For example, diabetics benefit from such nutrients as chromium, carnitine, taurine and vitamin E".
D2* nennt als Anwendungsbereich die Magen-Darm-Erkrankungen.

### Dokument D3*: WO 98/41113 A2 (SIGMA-TAU INDUSTRIE)

Das in Dokument D3* angegebene Ziel ist es, die therapeutische Behandlung des Typ 1 Diabetes und des Typ 2 Diabetes, die im wesentlichen auf der Anwendung von Insulin und oraler Antidiabetika beruht, durch eine angemessene Nahrungstherapie zu ergänzen. "Although ... the therapeutic treatment of type 1 and type 2 DM, based essentially on the administration of insulin and of oral hypoglycaemic agents, yield substantial efficiency, appropriate nutritional therapy is also of major importance ...." [S.2, Zeile 1]. Verwendet werden außer γ-Linolensäure und L-Carnitin zusätzlich noch Vitamine, Mineralien, Aminosäuren und Fettsäuren.

D3* hat als Anwendungsbereich den Typ 1 Diabetes (immunologisch und idiopathisch) sowie den Typ 2 Diabetes.

## Patentansprüche

1. Verwendung von Substanzen zur Herstellung eines Arzneimittels für die Prophylaxe und Behandlung des L-Diabetes, wobei der L-Diabetes definiert wird als Teilmenge des nicht-autoimmunopathischen und nicht-adipösen Diabetes mellitus, die **gekennzeichnet ist durch** einen im Zentralnervensystem falsch niedrig gemessenen Ist-Wert der Blutglucose, und wobei das Gesamtarzneimittel eine Kombinationen ist aus sechs Teilarzneimitteln, die zum Teil selbst Stoffkombinationen-darstellen; deren Einzelstoffauswahl strikt am pathogenetischen Kausalkonzept des L-Diabetes orientiert ist, zur Besserung der dort deklarierten pathophysiologischen Zustände und wobei das erste Teilarzneimittel, eingesetzt zur Verbesserung der im pathogenetischen Kausalkonzept des L-Diabetes bedeutsamen schwefelverbrauchenden Konjugationen besteht aus
- N-Acetylcystein,
oder aus einer Auswahl aus der nachfolgenden Substanzenliste, oder aus einer Kombination von N-Acetylcystein und einer Auswahl aus der nachfolgenden Substanzenliste, wobei die Substanzenliste
- Glutathion,
- Täurin,
- L-Cystin,
- L-Methionin und
- S-Adenosylmethionin
enthält, und wobei das zweite Teilarzneimittel, eingesetzt zur Verringerung der im pathogenetischen Kausalkonzept des L-Diabetes bedeutsamen Radikalenwirkungen aus einer Auswahl aus der nachfolgenden Substanzenliste besteht, wobei die Substanzenliste
- Vitamin C,
- injizierbares Vitamin E-Präparat,
- α-Liponsäure,
- Selen,
- Mangan,
- Rutosid,
- Hesperidin,
- Silibinin und
- Silymarin
enthält, und wobei das dritte Teilarzneimittel, eingesetzt zur Verbesserung der im pathogenetischen Kausalkonzept des L-Diabetes bedeutsamen Minderversorgung mit Phospholipiden und Phospholipidkomponenten aus
- Lecithin
besteht, oder aus einer Auswahl aus der nachfolgenden Substanzenliste, oder aus einer Kombination von Lecithin und einer Auswahl aus der nachfolgenden Substanzenliste, wobei die Substanzenliste
- Kephalin,
- Phosphatidylinositol,
- Inositol,
- Cholin,
- Cholinorotat
- Cholinchlorid,
- Cholincitrat und
- Cholinhydrogentartrat
enthält, und wobei das vierte Teilarzneimittel, eingesetzt zur Verbesserung der im pathogenetischen Kausalkonzept des L-Diabetes bedeutsamen Minderversorgung mit fettlöslichen Vitaminen und essentiellen Fettsäuren aus möglichst vielen der Substanzen
- Vitamin A,
- Vitamin D,
- Omega-3-Fettsäuren und
- Omega-6-Fettsäuren
besteht, und wobei als Cholagogum zur Verbesserung der Fettresorption zusätzlich
- Extrakt aus Rhizoma Curcumae xanthorrhizae
gewählt werden kann, und wobei das fünfte Teilarzneimittel, eingesetzt zur Verringerung der im pathogenetischen Kausalkonzept des L-Diabetes bedeutsamen Verluste an Vitaminen und Spurenelementen infolge bakterieller Überbesiedlung des Dünndarms, möglichst viele der Substanzen
- injizierbares Vitamin B₁-Präparat,
- injizierbares Vitamin B₂-Präparat,
- injizierbares Vitamin B₆-Präparat,
- injizierbares Vitamin B₁₂-Präparat,
- injizierbares Folsäure-Präparat und
- Zn⁺⁺
enthält, und wobei das sechste Teilarzneimittel, eingesetzt zur Verbesserung der im pathogenetischen Kausalkonzept des L-Diabetes bedeutsamen entzündlich-atrophischen Dünndarmmukosa, aus
- Dexpanthenol
besteht.

2. Verwendung gemäß Anspruch 1 von Substanzen zur Herstellung eines Arzneimittels für die Therapie und/oder Prophylaxe der in Anspruch 1 genannten Erkrankung, mit der Änderung, daß ergänzend beliebige Arzneimittel oder Wirkstoffe hinzugefügt werden.

## Claims

1. The use of substances for manufacturing a medication for the prophylaxis and therapy of L-diabetes, whereby L-diabetes is defined as a part of the non-autoimmunopathic and non -adipose diabetes mellitus that is **characterized by** a falsely low registered blood glucose level in the central nervous system, and whereby the compound medication is a combination of six other partial medications which themselves may be combinations of agents, and whereby the choice of individual agents is oriented strictly towards the pathogenic concept of L-diabetes so that the pathophysiological condition declared there can be improved, and whereby the first medication component, applied to promote the sulphur utilizing conjugations implicated in the pathogenic concept of L-diabetes, consists of
• N-acetyl-cysteine
or a selection of substances from the following list, or a combination of N-acetyl-cysteine and a selection of substances from the following list, whereby the list contains
• glutathione
• taurine
• L-cystine
• L-methionine and
• S-adenosyl-methionine
and whereby the second medication component, applied to reduce the effects of free radicals implicated in the pathogenic concept ofL-diabetes, consists of a selection of substances from the following list, whereby the list contains
• vitamin C
• an injectable vitamin E preparation
• α liponic acid
• selenium
• manganese
• rutoside
• hesperidine
• silibinin and
• silymarin
and whereby the third medication component, applied to improve the reduced supply of phospholipids and phospholipids components implicated in the pathogenic concept of L-diabetes, consists of
• lecithin
or a choice of medications from the following list, or a combination of lecithin and a choice of medications from the following list, whereby the list contains
• cephalin
• phosphatidyl-inositol
• inositol
• choline
• choline orotate
• choline chloride
• choline citrate and
• choline hydrogen tartrate
and whereby the fourth medication component, applied to improve the reduced supply of fat soluble vitamins and essential fatty acids implicated in the pathogenic concept of L-diabetes, consists of as many of the following substances
• vitamin A
• vitamin D
• omega-3-fatty acids and
• omega-6-fatty acids
and whereby as a cholagogic in order to improve fat resorption an
• extract from rhizoma curcumae xanthorrhizae
can be chosen, and whereby as a fifth medication component, applied to reduce the loss in vitamins and trace elements subsequent to the bacterial overcolonisation of the small intestine implicated in the pathogenic concept of L-diabetes, consists of as many of the following substances
• injectable vitamin B₁ preparation
• injectable vitamin B₂ preparation
• injectable vitamin B₆ preparation
• injectable vitamin B₁₂ preparation
• injectable folic acid preparation
• Zn⁺⁺
and whereby the sixth medication component, applied to improve the inflammatory-atrophic small intestinal mucosa implicated in the pathogenic concept of L-diabetes, consists of
• dexpanthenol.

2. Use according to claim 1 of substances to manufacture a medication for the therapy and prophylaxis of the disease cited in claim 1, with the amendment that as many medications or active principles as desired may be added to it.

## Revendications

1. La découverte se **caractérise par** l'utilisation de substances pour la fabrication d'un médicament destiné à la prophylaxie et au traitement du diabète de type L, le diabète L étant défini comme une partie du diabète mellitus d'origine non-autoimmune et non-adipeux, **caractérisé par le fait qu'**au niveau du système nerveux central la concentration réelle de glucose dans le sang est mesurée de manière erronée comme étant faible et où le médicament général est une combinaison de six médicaments partiels qui représentent en partie eux-mêmes des combinaisons de substances dont le choix de substances particulières s'oriente strictement au concept causal pathogénétique du diabète de type L, destiné à améliorer les états déclarés pathophysiologiques et où le premier médicament partiel, utilisé pour améliorer les conjugaisons consommatrices de soufre importantes dans le concept causal pathogénétique du diabète de type L, est composé
- de N-acétylcystéine,
ou d'une sélection des substances de la liste suivante ou d'une combinaison de N-acétylcystéine et d'une sélection des substances de la liste suivante, où la liste des substances contient
- du glutathion,
- de la taurine,
- de la L-cystine,
- de la L-méthionine et
- de la S-adénosylméthionine,
et où le deuxième médicament partiel, utilisé pour diminuer les effets des radicaux libres importants dans le concept causal pathogénétique du diabète de type L, comprend une sélection des substances de la liste suivante, où la liste des substances est composée
- de vitamine C,
- d'une préparation de vitamine E injectable,
- d'acide α-lipoïque,
- de sélénium,
- de manganèse,
- de rutoside,
- d'hespéridine,
- de silybine et
- de silymarine,
et où le troisième médicament partiel, utilisé pour améliorer la sous-alimentation en phospholipides et composants de phospholipides, importante dans le concept causal pathogénétique du diabète de type L, est composé
- de lécithine
ou d'une sélection des substances de la liste suivante ou d'une combinaison de lécithine et d'une sélection des substances de la liste suivante, où la liste des substances est composée
- de céphaline,
- de phosphatidyl-inositol,
- d'inositol,
- de choline,
- de cholinorotate
- de chlorure de choline,
- de citrate de choline et
- d'hydrogénotartrate de choline,
et où le quatrième médicament partiel, utilisé pour améliorer la sous-alimentation en vitamines liposolubles et acides gras essentiels, importante dans le concept causal pathogénétique du diabète de type L, est composé d'un maximum des substances suivantes
- vitamine A,
- vitamine D,
- acides gras oméga 3 et
- acides gras oméga 6,
et où on peut choisir en plus comme cholagogue pour l'amélioration de la résorption des graisses
- de l'extrait du rhizome Curcumae xanthorrhizae,
et où le cinquième médicament partiel, utilisé pour diminuer les pertes, importantes dans le concept causal pathogénétique, de vitamines et oligo-éléments à la suite de la colonisation bactérienne de l'intestin grêle, contient un maximum des substances
- préparation de vitamine B₁ injectable,
- préparation de vitamine B₂ injectable,
- préparation de vitamine B₆ injectable,
- préparation de vitamine B₁₂ injectable,
- préparation d'acide folique injectable et
- du Zn⁺⁺,
et où le sixième médicament partiel, utilisé pour améliorer la muqueuse inflammatoire et atrophiée de l'intestin grêle dans le concept causal pathogénétique du diabète L est composé
- de dexpanthénol.

2. Utilisation selon la revendication 1 de substances pour la fabrication d'un médicament destiné à la thérapie et/ou la prophylaxie de la maladie mentionnée en revendication 1, modifiée par l'adjonction d'un certain nombre de médicaments ou d'agents complémentaires.
